# EUROPÄISCHE PATENTSCHRIFT

(11) **EP 3 225 890 B1**
(45) Veröffentlichungstag und Bekanntmachung des Hinweises auf die Patenterteilung: **30.12.2020**
(21) Anmeldenummer: 17161951.3
(22) Anmeldetag: 14.07.2010
(51) Int. Cl.: F16K 11/085, F16K 27/06, A61M 39/22, A61M 39/16

(54) **MEHRWEGHAHN UND VERFAHREN ZU DESSEN HERSTELLUNG**
MULTI-WAY VALVE AND METHOD OF PRODUCING THE SAME
SOUPAPE À VOIES MULTIPLES ET SON PROCÉDÉ DE FABRICATION

(30) Priorität: 14.07.2009 DE 102009026172
(43) Veröffentlichungstag der Anmeldung: 04.10.2017
(62) Dokumentnummer(n) der früheren Anmeldung(en) nach Art. 76 EPÜ: 10742107.5 / 2 454 506
(73) Patentinhaber: Fresenius Kabi Deutschland GmbH, 61352 Bad Homburg (DE)
(72) Erfinder: HOPF, Hans-Jürgen, 90513 Zirndorf (DE); KASSAI, Norbert, 90522 Oberasbach (DE); HOPF, Alexander, 90491 Nürnberg (DE); HOPF, Michael, 90513 Zirndorf (DE)
(74) Vertreter: Fresenius Kabi Deutschland GmbH

(56) Entgegenhaltungen:
- WO-A1-2004/031635
- DE-U1- 20 005 691
- DE-U1-202010 004 098
- GB-A- 1 218 955
- GB-A- 1 344 166

## Beschreibung

Die vorliegende Erfindung betrifft einen Mehrweghahn, insbesondere 2-, 3- und 4-Weghähne wie sie insbesondere in der Medizin eingesetzt werden und ein Verfahren zur Herstellung eines solchen Mehrweghahns.

Mehrweghähne insbesondere 2-, 3- oder 4-Weghähne sind im Stand der Technik bekannt und werden insbesondere in der Medizin und Medizintechnik eingesetzt. Hierbei werden sie insbesondere im Infusionsbereich, dem Bereich der künstlichen Ernährung, bei der Transfusion und insbesondere für die Zu- bzw. Überleitung von verschiedenen Durchflussmedien und als sogenanntes "Injektions-Equipment" für die medizinische und pharmazeutische Ausrüstung verwendet. Die Mehrweghähne werden ferner auch in medizinischen Systemen verwendet, welche unter anderem aus mehreren Komponenten bestehen. Solche Systeme umfassen unter anderem Schwerkraftinfusionen, Pumpen oder Pumpüberleitungssysteme, Sondennahrungssysteme, Injektionen, Kombinationen hiervon und dergleichen.

Ein Mehrweghahn kann ferner durch die Kombination mit mehreren Mehrweghähnen zu einer sog. Mehrwegehahnbank oder "Manifold" (Mehrfachverteiler) montiert werden.

Bei der Bereitstellung von Mehrweghähnen ist von Bedeutung, dass insbesondere eine vorgegebene Dichtheit, eine leichte Bedienungsanwendung und eine Gratfreiheit der Bauteile gegeben ist. Insbesondere ist hierbei zu beachten, dass Mehrweghähne zur Zuführung unterschiedlicher Medien verwendet werden, welche unter anderem sehr unterschiedliche Viskositäten aufweisen.

In den im Stand der Technik bekannten Mehrweghähnen ist es unter anderem von Nachteil, dass diese in verschiedenen Anwendungsfällen Schwächen aufweisen, die unter anderem die Dichtheit, die Bedienerfreundlichkeit und Probleme beim Durchfluss verschieden viskoser Medien sind.

Darüber hinaus haben die im Stand der Technik bekannten fluiddurchströmte Mehrweghähne auch den Nachteil, dass diese unter anderem aus Kunststoffen hergestellt werden, bei deren Verarbeitung Weichmacher, wie beispielsweise Bisphenol-A eingesetzt werden. Bei der Verwendung der fluiddurchströmten Mehrweghähnen kann jedoch nicht ausgeschlossen werden, dass Teile der Weichmacher an die Flüssigkeit abgegeben werden, wobei bekannt ist, dass diese Weichmachermaterialen zu einer Belastung der körperlichen Gesundheit führen können. Unter anderem werden diesen Weichmachern Auswirkungen auf den Hormonhaushalt beziehungsweise kanzerogene Wirkungen zugeschrieben.

Ein weiterer Nachteil der im Stand der Technik bekannten Fluidverbindungen ist, dass diese im Gebrauch zum Teil mehr oder weniger offen platziert werden, so dass diese insbesondere wenn dem Patienten keine Flüssigkeit appliziert wird, mit der Luft im Behandlungsraum bzw. sogar in Kontakt mit anderen Gegenständen und Personen gelangen und dadurch zum Beispiel mit Keimen verunreinigt werden. Diese Kontamination kann unter Umständen zu schwerwiegenden Infektionen bei den zu behandelnden Personen führen.

Aus der GB 1 344 166 A ist ein Mehrweghahn bekannt, bei der ein Stellglied nach unten offene Tore aufweist und in einer Stellgliedaufnahme eines Gehäuses verdrehbar angeordnet ist. Am Boden der Stellgliedaufnahme ist eine Erhebung gebildet, mit der das Stellglied in Eingriff steht.

Aus der WO 2004/031635 A1 ist ein Zweiwegehahn bekannt, bei der ein Stellglied mit einem Endabschnitt in einen Lagerabschnitt einer Stellgliedaufnahme eingreift.

Die GB 1 218 955 A1 beschreibt einen Mehrweghahn, bei dem ein Stellglied mit einem Endabschnitt in eine zugehörige Lageraufnahme am Boden einer Stellgliedaufnahme eingreift.

Aufgabe der vorliegenden Erfindung ist es, die im Stand der Technik bekannten Nachteile wenigstens teilweise zu überwinden.

Die vorstehende Aufgabe wird durch einen erfindungsgemäßen Mehrweghahn gemäß Anspruch 1 gelöst. Die Aufgabe wird ferner auch durch ein Verfahren zur Herstellung eines entsprechenden Mehrweghahns sowie der Verwendung eines Mehrweghahns in der Medizin und/oder Medizintechnik gelöst. Ferner wird die Aufgabe auch durch die Verwendung entsprechender Materialien für die Herstellung zumindest der mit dem Fluid in Kontakt tretenden Oberflächen gelöst. Bevorzugte Ausgestaltungsformen sowohl des Mehrweghahns, als auch des Verfahrens zur Herstellung des Mehrweghahns sind Gegenstand der entsprechenden Unteransprüche.

Der erfindungsgemäße Mehrweghahn zum Einsatz in der Medizin und/oder Medizintechnik weist ein wenigstens abschnittsweise von einem Medium durchströmbares Grundgehäuse und ein in dem Grundgehäuse um eine zentrale Achse drehbeweglich aufgenommenes Stellglied auf. Der erfindungsgemäße Mehrweghahn ist dadurch gekennzeichnet, dass er Anschlussstellen für den Zulauf und den Ablauf des Mediums aufweist.

Des Weiteren bildet das Grundgehäuse des erfindungsgemäßen Mehrweghahns eine Stellgliedaufnahme, welche dadurch gekennzeichnet ist, dass sie einen konzentrisch zur zentralen Achse des Grundgehäuses angeordneten Zapfen, sowie eine an der Innenseite des Grundgehäuses radial umlaufende Aussparung ausweist. Das Stellglied des erfindungsgemäßen Mehrweghahns weist ein Bedienelement, sowie einen im Wesentlichen hohlzylindrischen Abschnitt auf, der den Zapfen der Stellgliedaufnahme des Grundgehäuses wenigstens abschnittsweise aufnimmt, wenigstens eine Durchflussöffnung zur Fluidverbindung wenigstens zweier Anschlussstellen und an der Außenseite des hohlzylindrischen Abschnitts wenigstens eine radial umlaufende Ausformung aufweist.

Der Zapfen des Grundgehäuses des erfindungsgemäßen Mehrweghahns ist so ausgebildet, dass er wenigstens im Bereich einer Anschlussstelle des Grundgehäuses ein Gegenlager für das Stellglied aufweist beziehungsweise bereitstellt, welches zusammen mit dem Stellglied eine Dichtfläche im Bereich wenigstens einer Anschlussstelle bildet. Der erfindungsgemäße Mehrweghahn ist dadurch gekennzeichnet, dass der Zapfen des Grundgehäuses wenigstens eine Abflachung am Umfang zur zentralen Achse aufweist und die Ausformung des Stellglieds in die Aussparung des Stellgliedaufnahme des Grundgehäuses zur fromschlüssigen und vorzugsweise fluiddichten Fixierung des Stellglieds in der Stellgliedaufnahme eingreift.

Unter Medium im Sinne der vorliegenden Erfindung werden alle Arten von Fluiden, insbesondere Flüssigkeiten, wie beispielsweise wässrige Flüssigkeiten, Blut, Ernährungsbrei, etc. und auch Gase verstanden.

Unter dem Grundgehäuse im Sinne der vorliegenden Erfindung wird ein Bauteil des Mehrweghahns, welcher das Gehäuse mit den Anschlussstellen und der Stellgliedaufnahme aufweist verstanden, wobei unter letzterer der Bereich des Grundgehäuses verstanden wird, welcher zur Aufnahme des Stellglied dient.

Unter Stellglied im Sinne der vorliegenden Erfindung wird ein, wenigstens eine Strömungsöffnung abschließendes sogenanntes "Kücken" verstanden. Das Bedienelement im Sinne der vorliegenden Erfindung ist ein mit dem Stellglied fest verbundenes Element, wie zum Beispiel ein Hahn, mit dem sich das Stellglied um die zentrale Achse drehen lässt. Dieser kann einen oder mehrere, üblicherweise zwei, drei oder vier Stellhebel aufweisen.

Unter einer Abflachung am Umfang des Zapfens wird eine Veränderung des kreisrunden Umfangs des Zapfens in Form einer Kreissehne in der Draufsicht verstanden, wobei die Abflachung eine entsprechende Fläche bildet.

In einer weiteren Ausführungsform des erfindungsgemäßen Mehrweghahns, weist das Grundgehäuse zwei, drei oder vier Anschlussstellen auf.

Gemäß einer weiteren, besonders bevorzugten Ausführungsform ist das den Mehrweghahn durchströmende Medium Flüssigkeiten, insbesondere ausgewählt aus einer Gruppe, welche Injektionslösungen, Infusionslösungen, Nährlösungen, Blut, Plasma sowie Kombinationen hieraus und dergleichen aufweist.

Gemäß einer weiteren, besonders bevorzugten Ausführungsform weist die den Mehrweghahn durchströmende Flüssigkeit eine Viskosität auf, welche 0,7 mPa s⁻¹ und 10⁶ mPa s⁻¹, bevorzugt zwischen 1 mPa s⁻¹ und 10⁵ mPa s⁻¹ und besonders bevorzugt bei etwa 10² mPa s⁻¹ liegt.

Gemäß einer weiteren Ausführungsform des erfindungsgemäßen Mehrweghahns, ist der Zapfen des Gründgehäuses wenigstens abschnittsweise konisch ausgeführt. In einer weiteren, besonders bevorzugten Ausführungsform sind konisch und nicht konische und/oder konische Abschnitte mit verschiedenen Konuswinkel in stufiger Abfolge ausgeführt. Der Konuswinkel gemäß der vorliegenden Situation wird als Abweichung von der zentralen Achse verstanden; insbesondere werden positive und negative Winkel unter Konuswinkel verstanden. In einer weiteren, besonders bevorzugten Ausführungsform weist der Zapfen eine alternierende Folge positiver und negativer Konuswinkel auf. Im einer weiteren bevorzugten Ausführungsform weist der Zapfen relativ zur zentralen Achse nach innen oder außen versetzte, insbesondere konische Abschnitte auf.

Gemäß einer weiteren bevorzugten Ausführungsform ist die Innenseite des hohlzylindrischen Abschnitts wenigstens abschnittsweise konisch ausgeführt und ist insbesondere an den konischen Verlauf des Zapfen im Wesentlichen angepasst.

Gemäß der Vorliegenden Erfindung sind die Durchflussöffnungen des Stellglieds torförmig ausgeführt, insbesondere derartig ausgeführt, dass die Durchflussöffnung zum Ende des hohlzylindrischen Abschnitts des Stellglieds offen ist. Ferner wird insbesondere die Durchflussöffnung vorzugsweise in Abhängigkeit des zu verwendenden Fluids in ihrer Größe angepasst wobei ferner auch eine Kombination der Form des Gegenlagers wie beispielsweise konisch und rechteckig mit berücksichtigt wird.

Gemäß einer weiteren, besonders bevorzugten Ausführungsform des erfindungsgemäßen Mehrweghahns entspricht die Zahl der Durchflussöffnungen des Stellglieds der Zahl der Anschlussstellen am Grundgehäuse. So weißt zum Beispiel eine Ausführungsform mit zwei Anschlussstellen zwei Durchflussöffnungen auf. Gemäß einer weiteren bevorzugten Ausführungsform weist das Stellglied weniger Durchlassöffnungen auf, als Anschlussstellen am Grundgehäuse vorhanden sind, wobei wenigstens und insbesondere zwei Durchlassöffnungen bzw. als Tore ausgestaltete Durchlassöffnungen vorgesehen sind.

Gemäß einer weiteren, besonders bevorzugten Ausführungsform des erfindungsgemäßen Mehrweghahns weist der hohlzylindrische Abschnitt des Stellglieds zwei, drei oder vier radial zur zentralen Achse umlaufende Ausformungen auf und die Innensite der Stellgliedaufnahme weist eine entsprechende Zahl an Aussparungen auf.

Gemäß einer weiteren, besonders bevorzugten Ausführungsform des Mehrweghahns gemäß der vorliegenden Erfindung ist die Mitte der Abflachung des Zapfens in der Stellgliedaufnahme des Grundgehäuses um 30° bis 60°, insbesondere um 45° versetzt zur Anschlussstelle des Grundgehäuses angeordnet, wobei die seitlichen Ränder der Abflachung ± 20° ± 1°, vorzugsweise ± 15 ± 2° und über etwa ± 10° von der Mitte der Abflachung versetzt angeordnet sind.

Gemäß einer weiteren, besonders bevorzugten Ausführungsform des erfindungsgemäßen Mehrweghahns wird das Gegenlager für das Stellglied durch einen nicht abgeflachten Bereich des Zapfens gebildet. Die Modifikation der Gegenlageraufnahme durch die erfindungsgemäße eine oder mehrere Abflachungen am Zapfen führt zu einer Reduktion der Reibungsfläche zwischen Zapfen und Stellglied. Dadurch wird eine Leichtgängigkeit (Bedienerfreundlichkeit) des Mehrweghahns gewährleistet und gleichzeitig sichergestellt, dass der Hahn nicht durch das eingesetzte, insbesondere viskose Medium, aus seiner definierten, eingestellten Position verschoben werden kann.

Unter Gegenlager im Sinne der vorliegenden Erfindung wird ein Bereich der Stellgliedaufnahme verstanden, der den Druck des auf Stellglied, insbesondere bei in geschlossenem Zustand, aufnimmt, bzw. einer Verformung des Stellglieds unter dem Druck des Mediums entgegen wirkt.

Gemäß einer weitern, besonders bevorzugten Ausführungsform des Mehrweghahns gemäß der vorliegenden Erfindung, bilden die radial umlaufende Ausformung am hohlzylindrischen Abschnitt des Stellglieds und die Aussparung and der Innenseite der Stellgliedaufnahme eine Fügeverbindung, wobei die Fügeverbindung insbesondere einen Fügering am Gehäuse aufweist. Der Fügering weist gemäß einer weiteren bevorzugten Ausführungsform einen radial umlaufenden Dichtabschnitt und/oder einen Positionierabschnitt auf. Insbesondere ist die Fügeverbindung zwischen dem Stellglied und dem Grundgehäuse so gestaltet, dass durch die Verbindung der beiden Teile eine Dichtheit von größer als 4 bar erreicht wird.

Gemäß einer weiteren bevorzugten Ausführungsform des erfindungsgemäßen Mehrweghahns wird wenigstens das Gehäuse und/oder das Stellglied wenigstens teilweise aus einem Material hergestellt, das aus einer Gruppe ausgewählt ist, welche neben Copolyester weitere Materialien wie zum Beispiel duro- und thermoplastische Kunststoff und insbesondere Polyphenylensulfid, Polypropylen, Poly-1-buten, Polyvinylchlorid, Polyvinylidenchlorid, Polymethyl-metaacrylat, Polyacrylnitril, Polystyrol, Polysulfon, Polyacetal, Polyvinylalkohol, Polyvinylacetat, Ionomere, Fluorkunststoff, Polyethylen, Polyamid, insbesondere ein teilaromatisches Polyamid, Polycarbonat, Polyester, Copolyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurthan, Polyvinylchlorid, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen und Materialien, welche keine Weichmacher insbesondere kein Bisphenol A oder Phthalate enthalten, sowie Kombinationen hiervon und dergleichen umfasst.

Gemäß einer weiteren bevorzugten Ausführungsform ist der erfindungsgemäße Mehrweghahns dadurch gekennzeichnet, dass wenigstens die mit dem Fluid in Kontakt kommenden Oberflächen des Verbindungssystems, insbesondere die innenliegenden Oberflächen des Mehrweghahns wenigstens abschnittsweise aus einem Material hergestellt sind, welches antiseptische und/oder antimikrobielle Eigenschaften aufweist.

Solche antimikrobielle oder antiseptsche Materialen sind beispielsweise Materialien wie hochporöses Silber, ionenfrei hergestelltes Silber, Silberverbindungen und insbesondere MicroSilber, Metallionen freisetzende Verbindungen, Kombinationen hiervon und dergleichen. Vorzugsweise werden diese im Bereich der entsprechenden Oberflächen angeordnet, wobei gemäß einer weiteren besonders bevorzugten Ausführungsform die entsprechenden Materialien auch in dem Kunststoff, aus welchem dar Mehrwegehahn bzw. dessen Bestandteile hergestellt sind eingebunden sind. So kann insbesondere ein hochporöses Silber, welches vorzugsweise auch im Wesentlichen ionenfrei hergestellt ist, in den Kunststoff eingemischt werden, wobei hieraus dann die Komponenten des Mehrweghahns wenigstens abschittsweise hergestellt werden. Alternativ liegt es auch im Sinn der vorliegenden Erfindung die Oberflächen mit einem entsprechenden Material bzw. Materialkombination zu beschichten.

Gemäß einer weiteren besonders bevorzugten Ausführungsform der vorliegenden Erfindung wird ein amorpher Copolyester zur Herstellung des Mehrweghahns für die Medizin und Medizintechnik und dessen Verwendung insbesondere für Infussions- oder Transfussionsschläuche, Mehrwegehähne, Mehrfachverteiler, Injektionsequipment wie Nadeln, Zugänge oder ähnliches und Kombinationen hiervon verwendet. Dabei ist das System dadurch gekennzeichnet, dass wenigstens die mit dem Fluid in Kontakt kommenden Oberflächen des Verbindungssystems wenigstens abschnittsweise aus einem amorphen Copolyester hergestellt werden.

Gemäß einer weiteren, besonders bevorzugten Ausführungsform werden wenigstens teilweise Materialien für die Herstellung des erfindungsgemäßen Verbindungssystems verwendet, welche keine Weichmacher, insbesondere keine Phthalate und kein Bisphenol A enthalten.

Die Aufgabe der vorliegenden Erfindung wird ferner auch durch ein Verfahren zur Herstellung eines Mehrweghahns gelöst, welches wenigstens die folgenden Schritt aufweist. In einem vorzugsweise ersten Schritt der erfindungsgemäßen Verfahrens wird das Grundgehäuse sowie das Stellglied hergestellt, wobei die Herstellung vorzugsweise im Spritzgußverfahren erfolgt. Nach der Aufnahme des Grundgehäuses in eine Montagevorrichtung gemäß des erfindungsgemäßen Verfahrens, wird der hohlzylindrische Abschnitt des Stellglieds in die Stellgliedaufnahme des Grundzylinders eingeführt und eingepresst. Der Einpressvorgang wird gemäß des erfindungsgemäßen Verfahrens mit Erreichen einer vorbestimmten Einpresstiefe beendet. Die Einpresstiefe wird insbesondere durch einen definierten Anschlag bestimmt. Die Dichtheit des erfindungsgemäßen Hahns bestimmt sich insbesondere durch die Definierte Einpresstiefe. So wird die Dichtheit im oberen Bereich durch die definierte Einpresstiefe in den Fügerand erreicht. Im inneren des Gehäuses wird die Dichtheit zu den entsprechenden Durchflussöffnungen über die Einpresstiefe und damit über das konische Gegenlager sichergestellt.

Gemäß einer weiteren, besonders bevorzugten Ausführungsform ist der die Einpresstiefe bestimmende Anschlag Bestandteil der umlaufenden Ausformung des hohlzylindrischen Abschnitts des Stellglieds und/oder Bestandteil der Stellgliedaufnahme des Grundgehäuses; insbesondere ist der Anschlag Bestandteil der Fügeverbindung.

Gemäß einer weiteren besonders bevorzugten Ausführungsform ist der das Gegenlager bildende Zapfen wenigstens abschnittsweise hohl ausgeführt oder ist gemäß einer weiteren besonders bevorzugten Ausführungsform aus Vollmaterial hergestellt.

Gemäß einer weiteren, besonders bevorzugten Ausführungsform handelt es sich bei den beim Fügen der Bauteile gemäß des erfindungsgemäßen Verfahrens auftretenden Verformungen im Wesentlichen um elastische Verformungen.

Die vorliegende Erfindung umfasst ferner auch die Verwendung des erfindungsgemäßen Mehrweghahns in der Medizin und Medizintechnik, insbesondere für die Zu- und Überleitung von verschiedenen Durchflussmedien, insbesondere für die Schwerkraftinfusion, für Pumpüberleitungssysteme, Sondennahrungssysteme, Injektionen sowie Kombinationen hiervon und dergleichen.

Die Erfindung wird nachfolgen anhand eines bevorzugten Ausführungsbeispiels erläutert, wobei darauf hingewiesen wird, dass durch dieses Beispiel Abwandlungen beziehungsweise Ergänzungen wie sie sich für den Fachmann unmittelbar ergeben mit umfasst sind. Darüber hinaus stellt dieses bevorzugte Ausführungsbeispiel keine Beschränkung der Erfindung in der Art dar, dass Abwandlungen und Ergänzungen im Umfang der vorliegenden Erfindung liegen.

Dabei zeigen:
Fig. 1 eine Aufsicht auf ein Grundgehäuse eines erfindungsgemäßen Dreiweghahns;
Fig. 2 einen Querschnitt durch das Grundgehäuse aus Fig. 1 in Richtung der B-B-Schnittlinie;
Fig. 3 einen Querschnitt durch das Grundgehäuse aus Fig. 1 in Richtung der A-A- Schnittlinie;
Fig. 4 eine Frontalansicht des Grundgehäuses aus Fig. 1;
Fig. 5 eine Detaildarstellung der radial umlaufenden Aussparung der Stellgliedaufnahme des Grundgehäuses;
Fig. 6 ein Stellglied eines erfindungsgemäße Mehrweghahns;
Fig. 7 einen Teilquerschnitt durch das Grundgehäuse aus

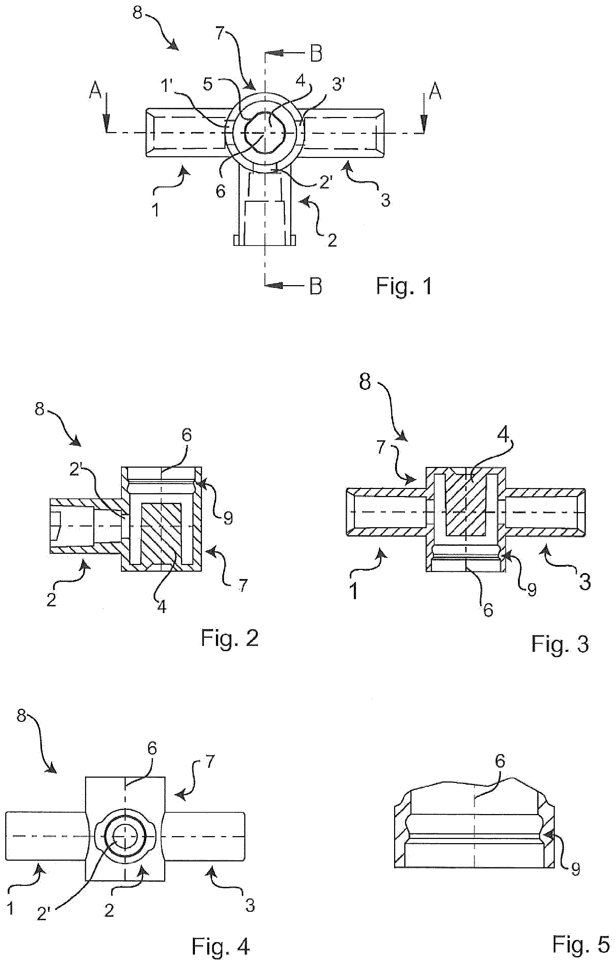

Fig. 1 in Richtung der A-A- Schnittlinie mit eingepresstem Stellglied;

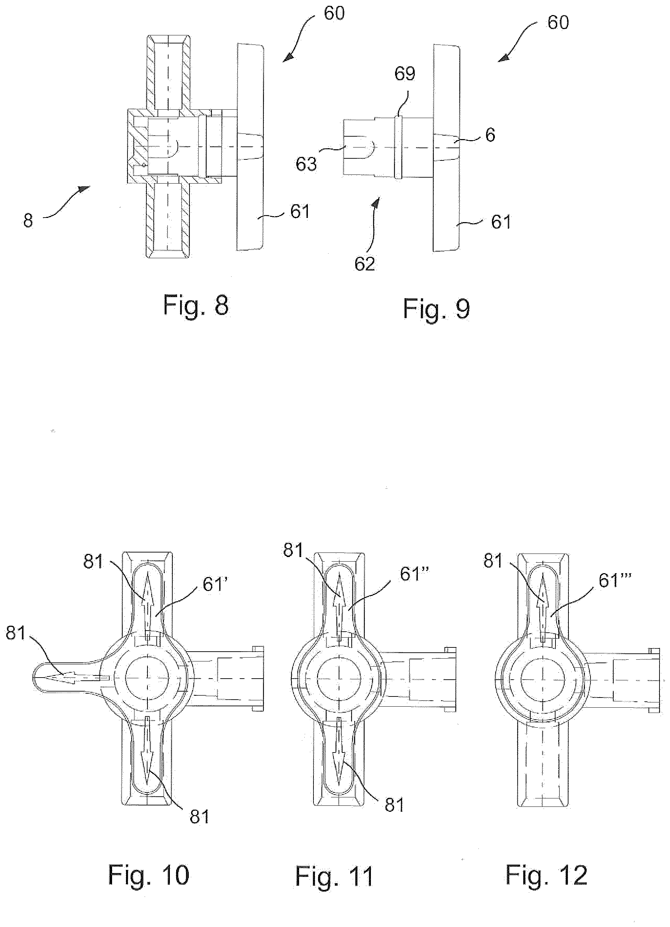

Fig. 8 bis 12 Seitenansicht und Draufsicht auf unterschiedlich ausgestaltete Mehrweghähne;

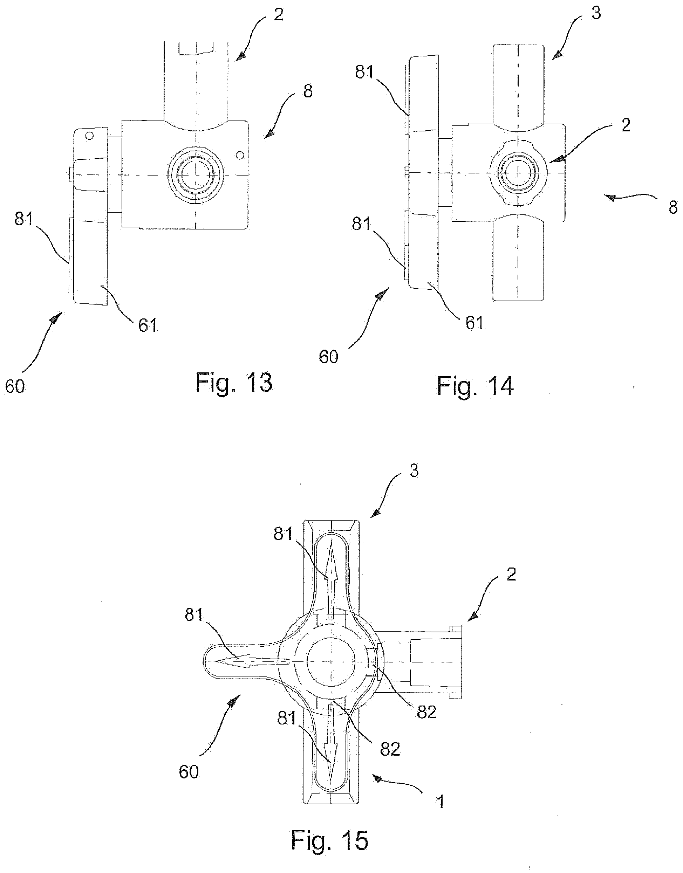

Fig. 13 bis 15 Seitenansicht und Draufsicht auf eine besonders bevorzugte Ausführungsform eines Mehrweghahns;

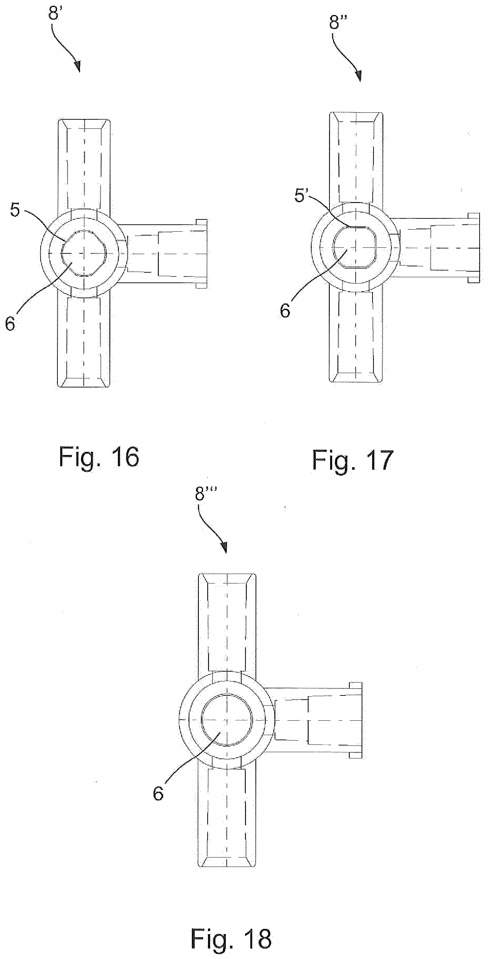

Fig. 16 bis 18 Draufsichten auf unterschiedlich ausgestaltete Grundgehäuse des erfindungsgemäßen Mehrweghahns.

Das Ausführungsbeispiel gemäß Figur 1 zeigt das Grundgehäuse 8 mit drei Anschlussstellen 1, 2, 3 und damit korrespondierenden Durchlassöffnungen 1', 2', 3' in der Stellgliedaufnahme 7. In der Stellgliedaufnahme 7 ist um eine zentrale Achse 6 ein Zapfen 4 mit vier Abflachungen 5 angeordnet. Die Unterbrochenen Linien markieren die Schnittlinien A-A und B-B für die Querschnitte in den Figuren 2 und 3.

So zeigt Figur 2 einen Querschnitt des Grundgehäuses in Richtung der B-B- Schnittlinie. In der Stellgliedaufnahme 6 ist um die zentrale Achse 7 der Zapfen 4 angeordnet. Die Anschlussstelle 2 ist mit der Stellgliedaufnahme verbunden, wobei die Durchlassöffnung 2' gebildet wird. Die Innenseite der Stellgliedaufnahme 6 weist eine radial Umlaufende Aussparung 9 auf, wobei die Aussparung 9 so gestaltet ist, dass sie unterschiedliche Tiefen aufweist.

Figur 3 zeigt einen Querschnitt des Grundgehäuses gemäß Figur 2 in Richtung der in Figur 1 markierten A-A- Schnittlinie. Die beiden Anschlussstellen 1 und 3 sind mit der Stellgliedaufnahme verbunden, wobei die Durchlassöffnungen 1' und 3' gebildet werden. Die Innenseite der Stellgliedaufnahme 6 weist eine radial Umlaufende Aussparung 9 auf, wobei die Aussparung 9 so gestaltet ist, dass sie unterschiedliche Tiefen aufweist.

Figur 4 zeigt eine Seitenansicht von vorn des Grundgehäuses entsprechend der Figur 1. Die Anschlussstellen 1, 2, 3 sind mit der Stellgliedaufnahme 6 verbunden. In der Mitte dieser Verbindungen befinden sich die entsprechenden Durchlassöffnungen, wie die Durchlassöffnung 2' in der Anschlussstelle 2.

Figur 5 zeigt einen Teil der Stellgliedaufnahme 6 mit der umlaufenden Aussparung 9 an ihrer Innenseite. Die Aussparung weist verschiedene Tiefen auf, so dass eine Abfolge von Vertiefungen und Auswölbungen bildet.

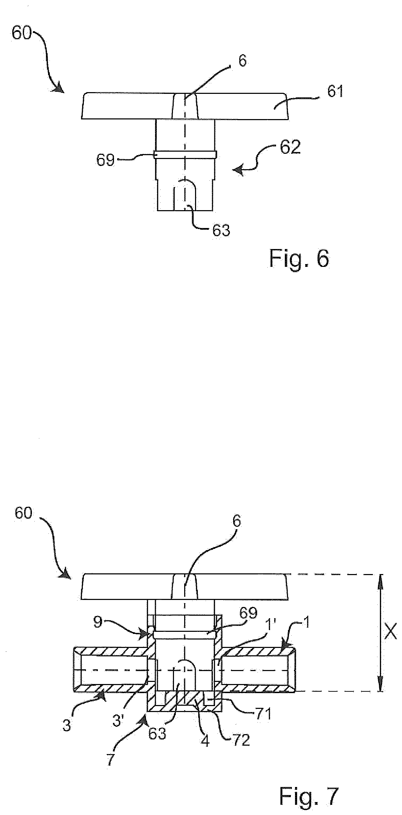

Figur 6 zeigt eine Ausführungsform eines Stellglied für die Verwendung in Verbindung mit dem Grundgehäuse gemäß Figur 1. Das Stellglied weist ein Bedienelement 61 in Form eines Hahnes mit drei Stellhebeln auf, welches fest mit einem hohlzylindrischen Abschnitt 62 verbunden ist. Der Hohlzylindrische Abschnitt ist konzentrisch um eine zentrale Achse 62 angeordnet. An dem Bedienelement gegenüberliegendem Ende des hohlzylindrischen Abschnitts sind torförmige Durchlassöffnungen 63 ausgebildet. Die Zahl der Durchlassöffnungen 63 des Stellglieds entspricht der Zahl der Anschlussstellen des Grundgehäuses. Am hohlzylindrischen Abschnitt 62 des Stellglieds 60 ist eine radial umlaufende Ausformung 69 angeordnet.

Figur 7 zeigt die Verbindung des Grundgehäuses (schraffiert dargestellt) entsprechend Figur 1 mit dem Stellglied 60 entsprechend der Figur 6 als eine Ausführungsform des erfindungsgemäßen Mehrweghahns. Das Stellglied 60 ist bis zu einer vorbestimmten Einpresstiefe X in die Stellgliedaufnahme 7 des Grundgehäuses eingepresst. In dieser Position greift die Ausformung 69 des hohlyzylindrischen Abschnitts des Stellglieds 60 in die Aussparung 9 an der Innenseite der Stellgliedaufnahme 6 des Grundgehäuses. Der hohlzylindrische Abschnitt des Stellglieds 60 umfasst den um eine zentrale Achse 6 angeordnete Zapfen 4 der Stellgliedausnahme 6 und bildet so ein Gegenlager für das Stellglied 60. Der Durchfluss des Mediums im geöffneten Zustand des Mehrweghahns erfolgt von einer der Anschlussstellen 1, 3 durch die zugehörige Durchlassöffnung des Grundgehäuses 1', 3' durch eine torförmige Durchlassöffnung des Stellglieds 63 über einen Freiraum 71 zwischen dem Stellglied 60 und dem Boden 72 der Stellgliedaufnahme 6 hin zur einer anderen Durchlassöffnung 63 des Stellglieds und durch die jeweils andere Durchlassöffnung 1', 3' des Grundgehäuses aus der jeweils anderes Anschlussstelle 1, 3 am Grundgehäuses hinaus. Die Dichtheit im oberen Bereich wird durch eine definierte Einpresstiefe des Stellglieds 60 in den Fügerand erreicht. Im Inneren des Grundgehäuses 8 wird die Dichtheit zu den entsprechenden Durchlassöffnungen 1, 2, 3 über das konische Gegenlager, gebildet durch den Zapfen 4, sichergestellt. Die Leichtgängigkeit des Hahns, also die leichte Beweglichkeit des Stellglieds 60 in der Stellgliedaufnahme 7 wird durch vier Abflachungen 5 am Zapfen 4 sichergestellt.

Die Figuren 8 bis 12 zeigen je eine Seitenansicht in Figur 8 und 9 und je eine Draufsicht in den Figuren 10 bis 12 von unterschiedlich ausgestaltete Mehrweghähne, bei welchen insbesondere die Bedienelemente 61', 61", 61''' unterschiedlich ausgestaltet sind. So zeigt Figur 10 ein dreiarmiges Bedienelement 61', Figur 11 ein zweiarmiges Bedienelement 61" und Figur 12 ein einarmiges Bedienelement, wie es insbesondere in Kombination mit unterschiedlich ausgestalteten Grundgehäusen 8 verwendet werden kann. Die auf dem Bedienelement angebrachten Pfeile 81 dienen ferner der leichteren Orientierung beim Einsatz der Mehrweghähne in der Praxis, um schematisch die Flussrichtung des Fluids anzugeben.

Die Figuren 13 bis 15 zeigen eine weitere Ausgestaltungsform in zwei Seitenansichten (Fig. 13 und 14) und in der Draufsicht (Fig. 15). Die wesentlichen Elemente sind dabei entsprechend den vorstehenden Abbildungen wieder gegeben. Darüber hinaus zeigt die hier dargestellte Ausführungsform ein Stellglied 60, bei welchem im hohlzylindrischen Abschnitt 62 (nicht dargestellt) nur zwei Durchgänge 82 (Tore) vorgesehen sind, die insbesondere zueinander mit einem Winkel von 90° angeordnet sind. Gemäß dieser Anordnung werden je nach Schaltung die Anschlüsse 1 und 2 oder 2 und 3 fluidverbunden. Eine Verbindung zwischen dem Anschluss 3 und 1 ist nicht möglich.

Die Figuren 16 bis 17 zeigen Draufsichten auf unterschiedlich ausgestaltete Grundgehäuse des erfindungsgemäßen Mehrweghahns, wobei sich hierbei insbesondere die zentrale Achse 6 in ihrer Ausgestaltung unterscheidet. So zeigt Figur 16 eine zentrale Achse mit vier Abflachungen 5, Figur 17 eine zentrale Achse 6 mit drei Abflachungen 5 und Figur 18 eine Zentrale Achse 6 ohne Abflachungen. Dabei ist zu berücksichtigen, dass insbesondere die Ausführungsform gemäß Figur 16 für parenterale Anwendungen geeignet ist und die Ausführungsform gemäß Figur 17 insbesondere für die Verwendung mit großen Schläuchen bzw. größeren Volumendurchsätzen geeignet ist.

## Patentansprüche

1. Mehrweghahn für den Einsatz in der Medizin oder Medizintechnik, mit wenigstens einem abschnittsweise von einem Medium durchströmbaren Grundgehäuse (8) und einem hierin, um eine zentrale Achse (6) drehbeweglich aufgenommenen Stellglied (60), wobei
das Grundgehäuse (8) wenigstens zwei Anschlussstellen (1, 2, 3) für den Zu- und Abfluss des Mediums aufweist und eine Stellgliedaufnahme (7) bildet, welche mit den Anschlussstellen (1, 2, 3) korrespondierende Durchlassöffnungen (1', 2', 3'), einen konzentrisch zur zentralen Achse (6) angeordneten Zapfen (4) und an der Innenseite des Grundgehäuses (8) eine radial umlaufende Aussparung aufweist (9), und
das Stellglied (60) ein Bedienelement (61) und einen im Wesentlichen hohlzylindrischen Abschnitt (62) aufweist, welcher den Zapfen (4) der Stellgliedaufnahme (7) wenigstens abschnittsweise aufnimmt, wenigstens eine Durchflussöffnung (63) zur Fluidverbindung wenigstens zweier Anschlussstellen (1, 2, 3) und an der Außenseite des zylindrischen Abschnitts (62) wenigstens eine radial umlaufende Ausformung (69) aufweist, und
der Zapfen (4) der Stellgliedaufnahme (7) wenigstens ein Gegenlager für das Stellglied (60) zur Bildung einer Dichtfläche im Bereich wenigstens einer Anschlussstelle (1, 2, 3) aufweist,
**dadurch gekennzeichnet, dass**
die Ausformung (69) des Stellglieds in die Aussparung (9) der Stellgliedaufnahme (7) zur formschlüssigen und fluiddichten Fixierung des Stellglieds (60) in der Stellgliedaufnahme (7) eingreift,
die Durchflussöffnungen (63) des Stellglieds (60) als Tore ausgeführt sind, welche zum Ende des hohlzylindrischen Abschnitts (62) des Stellglieds offen sind und
der Zapfen (4) sich vom Boden (72) der Stellgliedaufnahme (7) über die Durchlassöffnungen (1', 2', 3') des Grundgehäuses (8) erstreckt.

2. Mehrweghahn gemäß Anspruch 1, **dadurch gekennzeichnet, dass** der Zapfen (4) wenigstens eine Abflachung (5) am Umfang zur zentralen Achse (6) aufweist.

3. Mehrweghahn gemäß Anspruch 1 oder 2, **dadurch gekennzeichnet, dass** der Durchfluss des Mediums in einer geöffneten Stellung des Mehrweghahns von einer der Anschlussstellen (1, 2, 3) durch eine zugehörige Durchlassöffnung (1', 2', 3') des Grundgehäuses (8) und durch eine Durchlassöffnung (63) des Stellglieds (60) über einen Freiraum (71) zwischen dem Stellglied (60) und einem Boden (72) der Stellgliedaufnahme (7) hin zu einer anderen Durchlassöffnung (63) des Stellglieds (60) und durch eine andere Durchlassöffnung (1', 2', 3') des Grundgehäuses (8) aus einer anderen der Anschlussstellen (1, 2, 3) hinaus erfolgt.

4. Mehrweghahn gemäß einem der Ansprüche 1 bis 3, **dadurch gekennzeichnet, dass** das Grundgehäuse (8) zwei, drei oder vier Anschlussstellen (1, 2, 3) aufweist.

5. Mehrweghahn gemäß einem der vorstehenden Ansprüche, **dadurch gekennzeichnet, dass** das Medium eine Flüssigkeit ist, die aus einer Gruppe ausgewählt wird, welche Injektionslösungen, Infusionslösungen, Nährlösungen, Blut, Plasma, Gase sowie Kombinationen hieraus aufweist.

6. Mehrweghahn gemäß Anspruch 5, **dadurch gekennzeichnet, dass** die Flüssigkeit eine Viskosität aufweist, welche zwischen 0,7 mPa s-1 und 106 mPa s-1, insbesondere zwischen 1 mPa s-1 und 105 mPa s-1, und besonders bevorzugt bei etwa 102 mPa s-1 liegt.

7. Mehrweghahn gemäß einem der vorstehenden Ansprüche, **dadurch gekennzeichnet, dass** der Zapfen (4) wenigstens abschnittsweise konisch, vorzugsweise in stufiger Abfolge und/oder mit unterschiedlichen Konuswinkeln ausgeführt ist.

8. Mehrweghahn gemäß einem der vorstehenden Ansprüche, **dadurch gekennzeichnet, dass** die Innenseite des zylindrischen Abschnitts (62) des Stellglieds (60) wenigstens abschnittsweise konisch ausgeführt ist und insbesondere dem konischen Verlauf des Zapfens (4) im Wesentlichen entspricht.

9. Mehrweghahn gemäß einem der vorstehenden Ansprüche, **dadurch gekennzeichnet, dass** die Zahl der Durchflussöffnungen (63) des Stellglieds (60) der Zahl der Anschlussstellen (1, 2, 3) im Grundgehäuse (8) entspricht.

10. Mehrweghahn gemäß einem der vorstehenden Ansprüche, **dadurch gekennzeichnet, dass** zwei, drei oder vier radial zur zentralen Achse umlaufende Ausformung (69) im hohlzylindrischen Abschnitt (62) des Stellglieds (60) und eine entsprechende Zahl an Aussparungen (9) in der Stellgliedaufnahme (7) vorgesehen sind.

11. Mehrweghahn gemäß einem der vorstehenden Ansprüche, **dadurch gekennzeichnet, dass** die Mitte der Abflachung (5) am Zapfen (4) um 30° bis 60° und insbesondere um etwa 45° versetzt zur Anschlussstelle angeordnet ist, und die Ränder der Ablachung ± 20° ± 1°, vorzugsweise ± 15 ± 2° und über etwa ± 10° von der Mitte der Abflachung (5) versetzt angeordnet sind.

12. Mehrweghahn gemäß einem der vorstehenden Ansprüche, **dadurch gekennzeichnet, dass** das Gegenlager für das Stellglied (60) durch einen nicht abgeflachten Bereich des Zapfens (4) gebildet wird.

13. Mehrweghahn gemäß einem der vorstehenden Ansprüche, **dadurch gekennzeichnet, dass** die umlaufende Ausformung (69) im hohlzylindrischen Abschnitt (62) des Stellglieds (60) und die Aussparung (9) in der Stellgliedaufnahme (7) eine Fügeverbindung bilden, welche insbesondere einen Fügering am Gehäuse aufweist.

14. Mehrweghahn gemäß Anspruch 13, **dadurch gekennzeichnet, dass** der Fügering einen radial umlaufenden Dichtabschnitt und/oder Positionierabschnitt aufweist.

15. Mehrweghahn gemäß einem der vorstehenden Ansprüche, **dadurch gekennzeichnet, dass** das wenigstens das Grundgehäuse (8) und/oder das Stellglied (60) wenigstens teilweise aus einem Material hergestellt sind, das aus einer Gruppe ausgewählt ist, welche neben Copolyester, insbesodere amorphe Copolyester, weitere Materialien wie zum Beispiel duro- und thermoplastische Kunststoff und insbesondere Polyphenylensulfid, Polypropylen, Poly-1-buten, Polyvinylchlorid, Polyvinylidenchlorid, Polymethyl-metaacrylat, Polyacrylnitril, Polystyrol, Polysulfon, Polyacetal, Polyvinylalkohol, Polyvinylacetat, lonomere, Fluorkunststoff, Polyethylen, Polyamid, insbesondere ein teilaromatisches Polyamid, Polycarbonat, Polyester, Copolyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurthan, Polyvinylchlorid, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen und Materialien, welche keine Weichmacher, insbesondere kein Bisphenol A oder Phthalate enthalten, sowie Kombinationen hiervon umfasst.

16. Verfahren zur Herstellung eines Mehrweghahns gemäß einem der vorstehenden Ansprüche mit den Schritten
- Herstellen des Grundgehäuses (8) und des Stellglieds (60) vorzugsweise im Spritzgussverfahren;
- Aufnahme des Grundgehäuses (8) und des Stellglieds (60) in eine Montagevorrichtung;
- Einführen und Einpressen des hohlzylindrischen Abschnitts (62) des Stellglieds (60) in die Stellgliedaufnahme (7);
- Beenden des Einpressvorgang mit Erreichen einer vorbestimmten Einpresstiefe (X), die insbesondere durch einen definierten Anschlag bestimmt wird.

17. Verfahren gemäß Anspruch 16, **dadurch gekennzeichnet, dass** der Anschlag Bestandteil der umlaufenden Ausformung (69) im hohlzylindrischen Abschnitt (62) des Stellglieds (60) und/oder der Aussparung (9) in der Stellgliedaufnahme (7) und insbesondere der Fügeverbindung ist.

18. Verfahren gemäß einem der Ansprüche 16 oder 17, **dadurch gekennzeichnet, dass** die Verformungen beim Fügen der Bauteile, insbesondere im Bereich der Fügeverbindung im Wesentlichen elastische Verformungen sind.

19. Verwendung eines Mehrweghahns gemäß einem der Ansprüche 1 bis 15 in der Medizin und/oder Medizintechnik, für die Zu- bzw. Überleitung von verschiedenen Durchflussmedien für die Schwerkraftinfusion, Pumpüberleitungssysteme, Sondennahrungssysteme, und/oder Injektionen.

## Claims

1. A multi-way valve for use in medicine or medical technology, with at least one basic housing (8) through which a medium can flow in sections, and received therein in a rotating manner about a central axis (6), there is an actuator (60),
wherein
the basic housing (8) has at least two connection points (1, 2, 3) for the inflow and outflow of the medium and forms an actuator receiving portion (7), which has, corresponding to the connection points (1, 2, 3), through-flow openings (1', 2', 3'); arranged concentrically in relation to the central axis (6), it has a pin (4); and, on the inside of the basic housing (8), it has a radially circumferential recess (9), and the actuator (60) has an operating element (61) and a substantially hollow cylindrical section (62), which receives the pin (4) of the actuator receiving portion (7) at least in sections, has at least one through-flow opening (63) for the fluidic connection of at least two connection points (1, 2, 3) and, on the outer side of the cylindrical section (62), has at least one radially circumferential molding (69), and the pin (4) of the actuator receiving portion (7) has at least one counter bearing for the actuator (60) to form a sealing surface in the region of at least one connection point (1, 2, 3),
**characterized in that**
the molding (69) of the actuator engages into the recess (9) of the actuator receiving portion (7) in order to fix the actuator (60) in the actuator receiving portion (7) in a positive-locking and fluid-tight manner,
the through-flow openings (63) of the actuator (60) are designed as gates, which are open at the end of the hollow cylindrical section (62) of the actuator and
the pin (4) extends from the base (72) of the actuator receiving portion (7) over the through-flow openings (1', 2', 3') of the basic housing (8).

2. The multi-way valve according to claim 1, **characterized in that** the pin (4) has at least one flattened portion (5) on the circumference in relation to the central axis (6).

3. The multi-way valve according to claim 1 or 2, **characterized in that** the flow of the medium in an open position of the multi-way valve takes place from one of the connection points (1, 2, 3) through an associated through-flow opening (1', 2', 3') of the basic housing (8) and through a through-flow opening (63) of the actuator (60) via a free space (71) between the actuator (60) and a base (72) of the actuator receiving portion (7) towards another through-flow opening (63) of the actuator (60) and through another through-flow opening (1', 2', 3') of the basic housing (8) from another of the connection points (1, 2, 3).

4. The multi-way valve according to one of claims 1 to 3, **characterized in that** the basic housing (8) has two, three or four connection points (1, 2, 3).

5. The multi-way valve according to one of the preceding claims, **characterized in that** the medium is a fluid, which is selected from a group which includes injection solutions, infusion solutions, feeding solutions, blood, plasma, gases, and combinations thereof.

6. The multi-way valve according to claim 5, **characterized in that** the fluid has a viscosity, which is between 0.7 mPa s-1 and 106 mPa s-1, in particular between 1 mPa s-1 and 105 mPa s-1, and particularly preferably is roughly 102 mPa s-1.

7. The multi-way valve according to one of the preceding claims, **characterized in that** the pin (4), at least in sections, is designed conically, preferably in stepped sequence and/or at different cone angles.

8. The multi-way valve according to one of the preceding claims, **characterized in that** the inner side of the cylindrical section (62) of the actuator (60), at least in sections, is designed conically and in particular essentially corresponds to the conical course of the pin (4).

9. The multi-way valve according to one of the preceding claims, **characterized in that** the number of through-flow openings (63) of the actuator (60) corresponds to the number of connection points (1, 2, 3) in the basic housing (8).

10. The multi-way valve according to one of the preceding claims, **characterized in that** two, three or four moldings (69), radially circumferential in relation to the central axis, are provided in the hollow cylindrical section (62) of the actuator (60) and a corresponding number of recesses (9) in the actuator receiving portion (7).

11. The multi-way valve according to one of the preceding claims, **characterized in that** the middle of the flattened portion (5) on the pin (4) is arranged offset by 30° to 60° and in particular by roughly 45° in relation to the connection point, and the edges of the flattened portion are arranged offset by ± 20° ± 1°, preferably ± 15 ± 2° and over roughly ± 10° from the middle of the flattened portion (5).

12. The multi-way valve according to one of the preceding claims, **characterized in that** the counter bearing for the actuator (60) is formed by a non-flattened region of the pin (4).

13. The multi-way valve according to one of the preceding claims, **characterized in that** the circumferential molding (69) in the hollow cylindrical section (62) of the actuator (60) and the recess (9) in the actuator receiving portion (7) form a joint which has in particular a joining ring on the housing.

14. The multi-way valve according to claim 13, **characterized in that** the joining ring has a radially circumferential sealing section and/or positioning section.

15. The multi-way valve according to one of the preceding claims, **characterized in that** at least the basic housing (8) and/or the actuator (60) are at least partially made of a material, which is selected from a group, which in addition to copolyester, includes in particular amorphous copolyester, further materials such as for example duroplastic and thermoplastic synthetic material and in particular polylphenyl sulphide, polypropylene, poly-1-butene, polyvinylchloride, polyvinylidene chloride, polymethyl metaacrylate, polyacryl nitre, polystyrene, polysulphone, polyacetal, polyvinyl alcohol, polyvinyl acetate, ionomers, fluoroplastic, polyethylene, polyamide, in particular a partially aromatic polyamide, polycarbonate, polyester, copolyester, polyphenyl oxide, polysulphone, polyvinyl acetal, polyurethane and chlorinated polyether, cellulose nitrate, cellulose acetate, cellulose ether, phenol resin, urea resin, thiourea resin, melamine resin, alkyl resin, allyl resin, silicone, polyimide, polybenzimidazole, epoxide resin, casein plastic, cross-linked polyurethane, polyvinyl chloride, unsaturated polyester resin, antimicrobial or antiseptic materials such as for example highly porous silver, ion-free silver, silver compounds and in particular micro silver, metal ion-releasing compounds and materials which do not contain softening agents, in particular no bisphenol A or phthalates as well as combinations thereof.

16. A method of producing a multi-way valve according to one of the preceding claims, comprising the steps:
- producing the basic housing (8) and the actuator (60), preferably in an injection-molding process;
- placing the basic housing (8) and the actuator (60) into an assembly device;
- inserting and pressing the hollow cylindrical section (62) of the actuator (60) into the actuator receiving portion (7);
- ending the pressing-in operation upon reaching a predetermined pressing-in depth (X), which is determined in particular by a defined stop.

17. The method according to claim 16, **characterized in that** the stop is part of the circumferential molding (69) in the hollow cylindrical section (62) of the actuator (60) and/or the recess (9) in the actuator receiving portion (7) and in particular the joint.

18. The method according to one of claims 16 or 17, **characterized in that** the deformations when joining the components, in particular in the region of the joint, are essentially elastic deformations.

19. A use of a multi-way valve according to one of claims 1 to 15 in medicine and/or medical technology, for the supply and/or transfer of different fluid media for gravity infusion, pump transfer systems, tube feeding systems and/or injections.

## Revendications

1. Vanne à voies multiples pour une utilisation en médecine ou en technologie médicale, avec au moins un logement de base (8) à travers lequel un milieu peut s'écouler par sections, et reçu dans celui-ci de manière rotative autour d'un axe central (6), un actionneur (60), dans laquelle
le logement de base (8) a au moins deux points de raccordement (1, 2, 3) pour l'entrée et la sortie du milieu et forme une partie de réception d'actionneur (7), qui a, correspondant aux points de raccordement (1, 2, 3), des ouvertures de passage (1', 2', 3') ; il a, disposée concentriquement par rapport à l'axe central (6), une broche (4) ; et il a, à l'intérieur du logement de base (8), un évidement radialement circonférentiel (9), et
l'actionneur (60) a un élément de commande (61) et une section cylindrique essentiellement creuse (62), qui reçoit la broche (4) de la partie de réception d'actionneur (7) au moins par sections, a au moins une ouverture de passage (63) pour le raccordement fluidique d'au moins deux points de raccordement (1, 2, 3) et, du côté externe de la section cylindrique (62), a au moins un moulage radialement circonférentiel (69), et
la broche (4) de la partie de réception d'actionneur (7) a au moins un contre-palier pour l'actionneur (60) pour former une surface d'étanchéité dans la région d'au moins un point de raccordement (1, 2, 3),
**caractérisée en ce que**
le moulage (69) de l'actionneur vient en prise avec l'évidement (9) de la partie de réception d'actionneur (7) afin de fixer l'actionneur (60) dans la partie de réception d'actionneur (7) en engagement positif et de manière étanche aux fluides,
les ouvertures de passage (63) de l'actionneur (60) sont conçues comme des portes, qui sont ouvertes à l'extrémité de la section cylindrique creuse (62) de l'actionneur et
la broche (4) s'étend depuis la base (72) de la partie de réception d'actionneur (7) sur les ouvertures de passage (1', 2', 3') du logement de base (8).

2. Vanne à voies multiples selon la revendication 1, **caractérisée en ce que** la broche (4) a au moins une partie aplatie (5) sur la circonférence par rapport à l'axe central (6).

3. Vanne à voies multiples selon la revendication 1 ou 2, **caractérisée en ce que** l'écoulement du milieu dans une position ouverte de la vanne à voies multiples a lieu depuis l'un des points de raccordement (1, 2, 3) à travers une ouverture de passage associée (1', 2', 3') du logement de base (8) et à travers une ouverture de passage (63) de l'actionneur (60) via un espace libre (71) entre l'actionneur (60) et une base (72) de la partie de réception d'actionneur (7) vers une autre ouverture de passage (63) de l'actionneur (60) et à travers une autre ouverture de passage (1', 2', 3') du logement de base (8) d'un autre des points de raccordement (1, 2, 3).

4. Vanne à voies multiples selon l'une des revendications 1 à 3, **caractérisée en ce que** le logement de base (8) a deux, trois ou quatre points de raccordement (1, 2, 3).

5. Vanne à voies multiples selon l'une des revendications précédentes, **caractérisée en ce que** le milieu est un fluide, qui est choisi dans un groupe qui comporte des solutions d'injection, des solutions de perfusion, des solutions d'alimentation, du sang, du plasma, des gaz, et des combinaisons de ceux-ci.

6. Vanne à voies multiples selon la revendication 5, **caractérisée en ce que** le fluide a une viscosité, qui est comprise entre 0,7 mPa s-1 et 106 mPa s-1, en particulier entre 1 mPa s-1 et 105 mPa s-1, et de manière particulièrement préférée est d'environ 102 mPa s-1.

7. Vanne à voies multiples selon l'une des revendications précédentes, **caractérisée en ce que** la broche (4), au moins par sections, est conçue de manière conique, de préférence en séquence échelonnée et/ou à différents angles de cône.

8. Vanne à voies multiples selon l'une des revendications précédentes, **caractérisée en ce que** le côté interne de la section cylindrique (62) de l'actionneur (60), au moins par sections, est conçu de forme conique et correspond en particulier essentiellement à la course conique de la broche (4).

9. Vanne à voies multiples selon l'une des revendications précédentes, **caractérisée en ce que** le nombre d'ouvertures de passage (63) de l'actionneur (60) correspond au nombre de points de raccordement (1, 2, 3) dans le logement de base (8).

10. Vanne à voies multiples selon l'une des revendications précédentes, **caractérisée en ce que** deux, trois ou quatre moulages (69), radialement circonférentielles par rapport à l'axe central, sont prévus dans la section cylindrique creuse (62) de l'actionneur (60) et un nombre correspondant d'évidements (9) dans la partie de réception d'actionneur (7).

11. Vanne à voies multiples selon l'une des revendications précédentes, **caractérisée en ce que** le centre de la partie aplatie (5) sur la broche (4) est disposé décalé de 30° à 60° et en particulier d'environ 45° par rapport au point de raccordement et les bords de la partie aplatie sont disposés décalés de ±20° ± 1°, de préférence ±15 ± 2° et plus d'environ ±10° par rapport au centre de la partie aplatie (5).

12. Vanne à voies multiples selon l'une des revendications précédentes, **caractérisée en ce que** le contre-palier de l'actionneur (60) est formé d'une région non aplatie de la broche (4).

13. Vanne à voies multiples selon l'une des revendications précédentes, **caractérisée en ce que** le moulage circonférentiel (69) dans la section cylindrique creuse (62) de l'actionneur (60) et l'évidement (9) dans la partie de réception d'actionneur (7) forment une jointure qui a en particulier une bague de jonction sur le logement.

14. Vanne à voies multiples selon la revendication 13, **caractérisée en ce que** la bague de jonction a une section d'étanchéité radialement circonférentielle et/ou une section de positionnement.

15. Vanne à voies multiples selon l'une des revendications précédentes, **caractérisée en ce qu'**au moins le logement de base (8) et/ou l'actionneur (60) sont au moins partiellement fait en un matériau, qui est choisi dans un groupe, qui en plus du copolyester, comporte en particulier le copolyester amorphe, d'autres matériaux tels que par exemple le matériau synthétique duroplastique et thermoplastique et en particulier le sulfure de polyphényle, le polypropylène, le poly-1-butène, le chlorure de polyvinyle, le chlorure de polyvinylidène, le polyméthacrylate de méthyle, le polyacryl nitre, le polystyrène, le polysulfone, le polyacétal, l'alcool polyvinylique, l'acétate de polyvinyle, les ionomères, le fluoroplastique, le polyéthylène, le polyamide, en particulier un polyamide partiellement aromatique, le polycarbonate, le polyester, le copolyester, l'oxyde de polyphényle, le polysulfone, l'acétal polyvinylique, le polyuréthane et le polyéther chloré, le nitrate de cellulose, l'acétate de cellulose, l'éther de cellulose, la résine phénolique, la résine d'urée, la résine de thiourée, la résine de mélamine, la résine d'alkyle, la résine d'allyle, le silicone, le polyimide, le polybenzimidazole, la résine époxyde, le plastique de caséine, le polyuréthane réticulé, le chlorure de polyvinyle, la résine polyester insaturée, les matériaux antimicrobiens ou antiseptiques tels que par exemple l'argent hautement poreux, l'argent exempt d'ion, les composés d'argent et en particulier le micro-argent, les composés libérant des ions métalliques et les matériaux qui ne contiennent pas d'agents adoucissants, en particulier pas de bisphénol A ou de phtalates ainsi que des combinaisons de ceux-ci.

16. Procédé de production d'une vanne à voies multiples selon l'une des revendications précédentes, comprenant les étapes :
- produire le logement de base (8) et l'actionneur (60), de préférence dans un procédé de moulage par injection ;
- placer le logement de base (8) et l'actionneur (60) dans un dispositif d'assemblage ;
- insérer et enfoncer la section cylindrique creuse (62) de l'actionneur (60) dans la partie de réception d'actionneur (7) ;
- terminer l'opération d'enfoncement après avoir atteint une profondeur d'enfoncement (X) prédéterminée, qui est déterminée en particulier par une butée définie.

17. Procédé selon la revendication 16, **caractérisé en ce que** la butée fait partie du moulage circonférentiel (69) de la section cylindrique creuse (62) de l'actionneur (60) et/ou de l'évidement (9) dans la partie de réception d'actionneur (7) et en particulier de la jointure.

18. Procédé selon l'une des revendications 16 ou 17, **caractérisé en ce que** les déformations lors de la jonction des composants, en particulier dans la région de la jointure, sont essentiellement des déformations élastiques.

19. Utilisation d'une vanne à voies multiples selon l'une des revendications 1 à 15 en médecine et/ou technologie médicale, pour la fourniture et/ou le transfert de différents milieux fluides pour perfusion gravitaire, les systèmes de transfert de pompe, les systèmes d'alimentation par sonde et/ou les injections.
